# EUROPÄISCHE PATENTANMELDUNG

(11) **EP 3 741 304 A2**
(43) Veröffentlichungstag der Anmeldung: **25.11.2020**
(21) Anmeldenummer: 20175803.4
(22) Anmeldetag: 20.05.2020
(51) Int. Cl.: A61B 17/00

(54) **EXTRAKTIONSEINRICHTUNG UND APPLIKATIONSVORRICHTUNG MIT EINER EXTRAKTIONSEINRICHTUNG**

(30) Priorität: 24.05.2019 DE 102019113982
(71) Anmelder: Medi-Globe GmbH, 83101 Rohrdorf-Achenmühle (DE)
(72) Erfinder: HOCHBURGER, Tobias, 83022 Rosenheim (DE)
(74) Vertreter: Hofstetter, Schurack & Partner

(57) **Zusammenfassung**

Die Erfindung betrifft eine Extraktionseinrichtung (10) zur chirurgischen Entnahme von Gewebe, mit zumindest einem Extraktionsbeutel (20), welcher wenigstens eine Öffnung (22) zum Einführen des Gewebes in wenigstens einen Beutelinnenraum (24) des zumindest einen Extraktionsbeutels (20) aufweist und mit zumindest einer Zugvorrichtung (40), welche mit dem zumindest einen Extraktionsbeutel (20) verbunden und zum Verformen des zumindest einen Extraktionsbeutels (20) unter Verengung der zumindest einen Öffnung (22) ausgebildet ist. Die zumindest eine Zugvorrichtung (40) umfasst voneinander verschiedene Markierungen (50, 52) zur Kennzeichnung verschiedener Seiten (30, 32) des zumindest einen Extraktionsbeutels (20). Ein weiterer Aspekt der Erfindung betrifft eine Applikationsvorrichtung (100) mit einer Extraktionseinrichtung (10).

## Beschreibung

Die Erfindung betrifft eine Extraktionseinrichtung zur chirurgischen Entnahme von Gewebe, mit zumindest einem Extraktionsbeutel, welcher wenigstens eine Öffnung zum Einführen des Gewebes in wenigstens einen Beutelinnenraum des zumindest einen Extraktionsbeutels aufweist und mit zumindest einer Zugvorrichtung, welche mit dem zumindest einen Extraktionsbeutel verbunden und zum Verformen des zumindest einen Extraktionsbeutels unter Verengung der zumindest einen Öffnung ausgebildet ist. Ein weiterer Aspekt der Erfindung betrifft eine Applikationsvorrichtung mit einer Extraktionseinrichtung.

Eine derartige Extraktionseinrichtung kann eingesetzt werden, um beispielsweise von Tumoren befallenes Gewebe im Körper eines Patienten zu bergen und das Gewebe schließlich zusammen mit der Extraktionseinrichtung aus dem Körper zu befördern. Das befallene Gewebe wird dabei im Extraktionsbeutel platziert und der Extraktionsbeutel mit der Zugvorrichtung verschlossen, bevor die Extraktionseinrichtung aus dem Körper befördert wird.

Aus der DE 44 05 831 A1 ist eine Einrichtung zum Einbringen von Organen, Organteilen oder Gewebekomplexen innerhalb des menschlichen oder tierischen Körpers und zur Extraktion derselben ganz oder in gezielt fragmentierten Stücken bekannt. Die Einrichtung ist ein Beutel mit mehreren Öffnungen in der Beutelwand, die alle oder nur teilweise schlauchförmig sein können. Der Beutel wird zunächst über eine natürliche oder künstlich angelegte Körperöffnung in den Körper des Patienten eingebracht und zur Aufnahme eines abgetrennten Gewebekomplexes über eine der Öffnungen darin entfaltet. Die Öffnungen werden dann durch Trokarhülsen nach extrakorporal gezogen und befestigt, sodass Instrumente ins Beutelinnere eingeführt werden können, mit denen der Gewebekomplex endoskopisch weiter behandelt wird. Näher zu untersuchende Gewebeteile davon werden über eine der Öffnungen hierzu nach extrakorporal gebracht.

Die EP 0 696 899 B1 beschreibt eine Vorrichtung zur Durchführung endoskopischer Operationen, mit einer flexiblen Hülle, die abgetrennte Teile aufnimmt. Die Hülle weist mindestens zwei Öffnungen auf, von denen wenigstens eine Öffnung distal und wenigstens eine andere Öffnung im proximalen Bereich angeordnet ist. Die Hülle besteht aus einem elastischen, sterilisierbaren, reißfesten Werkstoff, wie beispielsweise Nylon, wobei ein Teil der Hülle transparent bzw. durchsichtig ausgeführt ist. Die Hülle ist ballonartig auf eine vordefinierte Form aufblasbar bzw. insuffl ierbar.

Aufgabe der vorliegenden Erfindung ist es, eine Extraktionseinrichtung sowie eine Applikationsvorrichtung der eingangs genannten Art zu schaffen, welche zum Einsparen von Zeit bei einem medizinischen Eingriff beitragen.

Diese Aufgabe wird durch die Gegenstände der unabhängigen Patentansprüche gelöst. Vorteilhafte Weiterbildungen der Erfindung sind durch die Merkmale der abhängigen Patentansprüche, die folgende Beschreibung sowie die Figuren offenbart.

Ein erster Aspekt der Erfindung betrifft eine Extraktionseinrichtung zur chirurgischen Entnahme von Gewebe, mit zumindest einem Extraktionsbeutel, welcher wenigstens eine Öffnung zum Einführen des Gewebes in wenigstens einen Beutelinnenraum des zumindest einen Extraktionsbeutels aufweist und mit zumindest einer Zugvorrichtung, welche mit dem zumindest einen Extraktionsbeutel verbunden und zum Verformen des zumindest einen Extraktionsbeutels unter Verengung der zumindest einen Öffnung ausgebildet ist. Die chirurgische Entnahme des Gewebes kann insbesondere durch Endoskopie erfolgen. Durch das Verengen der Öffnung kann zumindest weitgehend unterbunden werden, dass das in dem Beutelinnenraum eingeführte Gewebe unbeabsichtigt aus dem Beutelinnenraum herausfällt.

Gemäß der Erfindung ist vorgesehen, dass die zumindest eine Zugvorrichtung voneinander verschiedene Markierungen zur Kennzeichnung verschiedener Seiten des zumindest einen Extraktionsbeutels umfasst. Dies ist von Vorteil, da durch die voneinander verschiedenen Markierungen eine vereinfachte Orientierung der Extraktionseinrichtung zur Entnahme des Gewebes im Rahmen eines medizinischen Eingriffs ermöglicht ist, wodurch bei dem medizinischen Eingriff Zeit gespart werden kann. Mittels der Markierungen kann eine korrekte Platzierung der Extraktionseinrichtung und damit des Extraktionsbeutels beispielsweise in einem Abdomen vereinfacht, insbesondere beschleunigt werden. Sofern die Extraktionseinrichtung beispielsweise Tüllen aufweist, so erleichtern die Markierungen eine Orientierung der Tüllen derart, dass letztere beim medizinischen Eingriff beispielsweise zu einer Bauchdecke ausgerichtet sein können. Anhand der Markierungen kann beispielsweise eine schnelle Unterscheidung zwischen einer Oberseite (Vorderseite) und einer Unterseite (Rückseite) des Extraktionsbeutels erfolgen. Die Markierungen können sich vorzugsweise farblich voneinander unterscheiden, wodurch die Markierungen besonders einfach hergestellt werden können. In vorteilhafter Weise können die Markierungen zusätzlich oder alternativ auch verschiedene Oberflächenstrukturen aufweisen, was beispielsweise bei schlechten Lichtverhältnissen während des medizinischen Eingriffs eine vereinfachte Orientierung ermöglicht. Ebenfalls denkbar ist es, dass die Markierungen zusätzlich oder alternativ voneinander verschiedene Beschriftungen aufweisen, wodurch eine besonders intuitive Erkennung der Orientierung des Extraktionsbeutels während des medizinischen Eingriffs ermöglicht ist. Es kann auch vorgesehen sein, dass die voneinander verschiedenen Markierungen zusätzlich oder alternativ beispielsweise fluoreszierend ausgebildet sind, und beispielsweise in verschiedenen Farben leuchten können, was ebenfalls zur vereinfachten Orientierung, insbesondere bei schlechten Lichtverhältnissen beitragen kann.

Die Zugvorrichtung kann den Extraktionsbeutel in dessen Umfangsrichtung an einem die Öffnung begrenzenden Randbereich des Extraktionsbeutels zumindest über einen überwiegenden Bereich des Extraktionsbeutels umschlingen, wodurch eine besonders gezielte Verengung der Öffnung sowie ein gezieltes Verformen des Extraktionsbeutels ermöglicht ist. Besonders bevorzugt kann die Zugvorrichtung den Extraktionsbeutel in dessen Umfangsrichtung an dem Randbereich vollständig Umschlingen, wodurch besonders wirksam ein etwaiges, unbeabsichtigtes Herausfallen des Gewebes aus dem Beutelinnenraum unterbunden werden kann. Denkbar ist zusätzlich oder alternativ, dass an dem die Öffnung begrenzenden Randbereich des Extraktionsbeutels beispielsweise zumindest ein Laschenelement ausgebildet ist, in welchem die Zugvorrichtung zumindest bereichsweise in Umfangsrichtung im Bereich der Öffnung geführt sein kann, wodurch einem etwaigen Versatz der Zugvorrichtung relativ zum Extraktionsbeutel entgegengewirkt werden kann. Des Weiteren kann zusätzlich oder alternativ vorgesehen sein, dass an dem die Öffnung begrenzenden Randbereich des Extraktionsbeutels eine Mehrzahl an Durchtrittsöffnungen vorgesehen ist, welche in Umfangsrichtung entlang dem Randbereich verteilt sein können und in welche die Zugvorrichtung eingeführt sein kann, wodurch die Zugvorrichtung besonders verliersicher an dem Extraktionsbeutel geführt gehalten sein kann.

Die Zugvorrichtung kann zusätzlich oder alternativ beispielsweise an einer Beutelwand des Extraktionsbeutels entlang geführt sein, wobei die Beutelwand vorzugsweise zwischen der Zugvorrichtung und der Durchgangsöffnung sowie dem Beutelinnenraum angeordnet sein kann. Dadurch kann wirksam eine etwaige Kontamination der Zugvorrichtung infolge eines Kontakts der Zugvorrichtung mit krankhaftem, im Beutelinnenraum aufgenommenem Gewebe vermieden werden.

In einer vorteilhaften Weiterbildung der Erfindung umfasst die zumindest eine Zugvorrichtung wenigstens ein Zugelement und wenigstens eine Ummantelung, welche das zumindest eine Zugelement umgibt. Dies ist von Vorteil, da durch die Ummantelung größere Flexibilität bei einer Wahl eines Werkstoffs des Zugelements besteht. So kann es bereits ausreichend sein, die Ummantelung aus einem biokompatiblen Werkstoff bereitzustellen.

In einer weiteren vorteilhaften Weiterbildung der Erfindung ist die wenigstens eine Ummantelung und/oder das wenigstens eine Zugelement mit den voneinander verschiedenen Markierungen versehen. Dies ist von Vorteil, da hierdurch auf besonders einfache Art und Weise eine verbesserte Orientierung der Extraktionseinrichtung bei der Entnahme des Gewebes im Rahmen eines medizinischen Eingriffs ermöglicht ist. Ein Versehen der Ummantelung mit den voneinander verschiedenen Markierungen ist beispielsweise vorteilhaft, da die Ummantelung einen größeren Außendurchmesser als das Zugelement aufweist und die Markierungen damit besonders großflächig und deutlich erkennbar an der Ummantelung ausgebildet sein können. Ein Versehen des Zugelements mit den voneinander verschiedenen Markierungen ist beispielsweise vorteilhaft, da hierdurch eine größere Flexibilität bei der Auswahl der Markierungen hinsichtlich deren Biokompatibilität besteht, zumal die Ummantelung einen Kontakt der am Zugelement angeordneten Markierungen beispielsweise mit Organen verhindern kann. Die Ummantelung kann bevorzugt zumindest bereichsweise aus transparentem Material gebildet sein.

In einer weiteren vorteilhaften Weiterbildung der Erfindung ist das zumindest eine Zugelement als Draht, insbesondere Nitinol-Draht, ausgebildet. Dies ist von Vorteil, da über einen Draht besonders große Zugkräfte aufgebracht werden können, wodurch die Öffnung besonders wirksam verengt werden kann. Das Zugelement kann insbesondere aus einem Formgedächtnismaterial, wie beispielsweise Nitinol gebildet sein, wodurch das Zugelement bzw. die Zugvorrichtung in deren unbelastetem Zustand (ohne Zugbeanspruchung) die Öffnung besonders weit geöffnet halten kann. Das Zugelement kann also bevorzugt als Nitinol-Draht ausgebildet sein.

In einer weiteren vorteilhaften Weiterbildung der Erfindung umfassen die verschiedenen Seiten eine Vorderseite und eine Rückseite des zumindest einen Extraktionsbeutels. Dies ist von Vorteil, da die Vorderseite und die Rückseite des Extraktionsbeutels dadurch zumindest im Wesentlichen optisch ähnlich ausgebildet sein können, wodurch der Extraktionsbeutel mit geringem Aufwand hergestellt werden kann. Die Herstellung des Extraktionsbeutels kann beispielsweise ein Verschweißen eines die Vorderseite bildenden ersten Seitenelements mit einem, die Rückseite bildenden, zweiten Seitenelement an jeweiligen Seitenrändern dieser Seitenelemente umfassen. Die Unterscheidung zwischen Vorderseite und Rückseite kann einfach anhand der Markierungen der Zugvorrichtung erfolgen.

In einer weiteren vorteilhaften Weiterbildung der Erfindung umfasst die Extraktionseinrichtung wenigstens eine Tülle, welche eine mit dem zumindest einen Extraktionsbeutel verbundene und einen Tülleninnenraum der wenigstens einen Tülle zumindest bereichsweise umgrenzende Tüllenwandung aufweist, wobei der Tülleninnenraum über wenigstens eine am Extraktionsbeutel ausgebildete Durchgangsöffnung in den wenigstens einen Beutelinnenraum mündet und wobei zumindest eine mit der Tüllenwandung verbundene Lasche sowie wenigstens ein, durch eine Laschenöffnung der zumindest einen Lasche geführtes Verengungselement, welches zum Verformen der zumindest einen Tüllenwandung und bereichsweisen Verengen des Tülleninnenraums ausgebildet ist, vorgesehen sind. Dies ist von Vorteil, da das Verengungselement somit über die Lasche geführt an der Tüllenwandung gehalten werden kann, ohne dass das Verengungselement in den Tülleninnenraum eintritt. Dadurch kann der Tülleninnenraum anhand des Verengungselements verengt werden, und dadurch ein Austreten von Gewebe oder Gewebeflüssigkeit aus dem in den Beutelinnenraum mündenden Tülleninnenraum an die Umgebung der Extraktionseinrichtung zumindest weitgehend unterbunden werden, wobei aufgrund der Führung des Verengungselements über die Lasche auf etwaige Löcher in der Tüllenwandung zum Führen des Verengungselements verzichtet werden kann. Nachteilig an derartigen Löchern ist, dass diese Löcher ein Austreten von Gewebe bzw. Gewebeflüssigkeit an die Umgebung begünstigen.

Zusätzlich oder alternativ kann die wenigstens eine Tülle eine Engstelle aufweisen, welche beispielsweise als bereichsweise Verjüngung der Tüllenwandung ausgebildet sein kann. Die Tülle kann an der Engstelle elastisch verformbar ausgebildet sein. Zusätzlich oder alternativ kann die Extraktionseinrichtung ein an der Tüllenwandung angeordnetes, elastisches Element, beispielsweise einen Gummiring umfassen. Das elastische Element kann bevorzugt in die Tüllenwandung integriert sein, wodurch ein unerwünschtes Lösen des elastischen Elements von der Tüllenwandung besonders einfach vermieden werden kann. Durch die elastisch verformbare Engstelle bzw. durch das elastische Element kann die Tüllenwandung beispielsweise während des medizinischen Eingriffs auf ein in den Tülleninnenraum eingeführtes Instrument (beispielsweise Trokar) gedrückt werden, und somit ein Abdichten des Tülleninnenraums beispielsweise gegenüber einem Austreten von Gewebe oder Gewebeflüssigkeit bewirken.

Die Engstelle kann bevorzugt an einem, dem zumindest einen Extraktionsbeutel zugewandten und damit beutelnahen Tüllenende der Tülle angeordnet sein. Dadurch kann verhindert werden, dass Gewebe bzw. Gewebeflüssigkeit aus dem Beutelinnenraum in den Tülleninnenraum gelangt.

Zusätzlich oder alternativ kann die wenigstens eine Tülle an einem, dem zumindest einen Extraktionsbeutel abgewandten und damit distalen Tüllenende verschlossen, beispielsweise zugeschweißt, sein. Dies ist von Vorteil, da die Tülle in diesem Fall bedarfsgerecht, beispielsweise am Tüllenende geöffnet werden kann. Sofern die Tülle beim medizinischen Eingriff nicht verwendet wird, so kann diese einfach verschlossen bleiben und muss dementsprechend beim medizinischen Eingriff oder nach diesem Eingriff nicht separat verschlossen werden. Die Tülle kann beispielsweise am Tüllenende bei Bedarf aufgeschnitten und dadurch geöffnet werden. Vorzugsweise kann der Tülleninnenraum am (distalen) Tüllende verjüngt sein, mit anderen Worten kann die Tüllenwandung am Tüllenende eine Tüllenendenverjüngung aufweisen. Zumindest an der Tüllenendenverjüngung (Verjüngung der Tüllenwandung am Tüllenende) kann die Tüllenwandung elastisch verformbar ausgebildet sein, sodass nach einem etwaigen Öffnen des Tüllenendes und Einführen eines Instruments (beispielsweise Trokar) eine sichere Abdichtung gegenüber dem Austreten von Gewebe oder Gewebeflüssigkeit erfolgen kann.

Zusätzlich oder alternativ zu dem Verengungselement kann der Tülleninnenraum auch beispielsweise anhand eines Clips verschlossen werden. Dies ist von Vorteil, da durch ein Verschließen des Tülleninnenraums anhand eines Clips eine besonders sichere Abdichtung des Tülleninnenraums erfolgen kann.

Zusätzlich oder alternativ zu dem Verengungselement kann die wenigstens eine Tülle auch mit sich selbst verknotet werden, wodurch ein besonders einfaches Verschließen des Tülleninnenraums erfolgen kann.

In einer weiteren vorteilhaften Weiterbildung der Erfindung ist die Tüllenwandung zwischen dem Tülleninnenraum und dem wenigstens einen Verengungselement angeordnet. Dies ist von Vorteil, da die Tüllenwandung somit den Tülleninnenraum von dem Verengungselement trennt, wodurch eine etwaige Kontamination des Verengungselements mit im Beutelinnenraum aufgenommenem Gewebe bzw. Gewebeflüssigkeit vermieden werden kann.

In einer weiteren vorteilhaften Weiterbildung der Erfindung ist das Verengungselement als Faden oder als Seil oder als Draht ausgebildet. Dies ist von Vorteil, da das Verengen des Tülleninnenraums durch die Ausgestaltung des Verengungselements als Faden, als Seil oder als Draht besonders einfach durch Zuziehen und damit eine Zugkraftbeaufschlagung des Verengungselements erfolgen kann. Hierzu kann das Verengungselement die Tüllenwandung umfangsseitig bevorzugt zumindest überwiegend, insbesondere vollständig, umschlingen.

In einer weiteren vorteilhaften Weiterbildung der Erfindung umfasst das wenigstens eine Verengungselement zumindest einen ersten Verengungselementbereich mit wenigstens einem Knoten, wobei der wenigstens eine Knoten verschiebbar an zumindest einem zweiten Verengungselementbereich des wenigstens einen Verengungselements aufgenommen ist und die zumindest eine Tüllenwandung durch eine Relativverschiebung zwischen dem wenigstens einen Knoten und dem zumindest einen zweiten Verengungselementbereich anhand des wenigstens einen Verengungselements verformbar und dabei der Tülleninnenraum zumindest bereichsweise verengbar ist. Dies ist von Vorteil, da durch die Relativverschiebung zwischen dem Knoten und dem zweiten Verengungselementbereich ein besonders einfaches Verengen des Tülleninnenraums ermöglicht ist. So kann beispielsweise der Knoten festgehalten und der zweite Verengungselementbereich relativ zum Knoten verschoben werden, um den Tülleninnenraum zu verengen (und die Tüllenwandung zu verformen). Der Knoten und der zweite Verengungselementbereich können bevorzugt zumindest in einer gemeinsamen Verengungsstellung, in welcher der Tülleninnenraum anhand des Verengungselements verengt sein kann, eine selbsthemmende Verbindung bilden. Dadurch kann in vorteilhafter Weise eine unerwünschte Erweiterung des Tülleninnenraums verhindert und damit ein etwaiges Austreten von Gewebe bzw. Gewebeflüssigkeit über den Tülleninnenraum an die Umgebung vermieden werden.

Ein Verschließen der wenigstens einen Tülle kann initial mittels eines in den Tülleninnenraum und damit in die Tülle eingeführten Instruments, beispielsweise in Form eines Trokars, erfolgen. Der Knoten kann nach einem Entfernen des Instruments einfach weiter geschoben und dabei zugezogen werden, oder bei Bedarf auch wieder geöffnet werden, falls beispielsweise ein anderes, größeres Instrument eingeführt werden soll um beispielsweise die Tülle final zu verschließen.

Im Gegensatz dazu kann ein konventioneller (auf einer konventionellen Knotentechnik basierender) Knoten nicht weitergeschoben werden, und muss beispielsweise für ein finales Verschließen der Tülle zuvor erst wieder mühsam aufgeknotet werden.

In einer weiteren vorteilhaften Weiterbildung der Erfindung umfasst die Extraktionseinrichtung wenigstens eine zweite Tülle, welche durch wenigstens ein zweites Verengungselement verformbar und bereichsweise verengbar ist, wobei das wenigstens eine zweite Verengungselement und das wenigstens eine Verengungselement voneinander verschiedene Markierungen aufweisen. Dies ist von Vorteil, da durch die verschiedenen Markierungen des Verengungselements und des zweiten Verengungselements ebenfalls eine verbesserte Orientierung erfolgen und dadurch Zeit beim medizinischen Eingriff eingespart werden kann. So kann beispielsweise das Verengungselement gelb gefärbt und das zweite Verengungselement blau gefärbt sein, um nur ein Beispiel zu nennen.

Ein weiterer Aspekt der Erfindung betrifft eine Extraktionseinrichtung zur chirurgischen Entnahme von Gewebe, mit zumindest einem Extraktionsbeutel, welcher wenigstens eine Öffnung zum Einführen des Gewebes in wenigstens einen Beutelinnenraum des zumindest einen Extraktionsbeutels aufweist und mit zumindest einer Zugvorrichtung, welche mit dem zumindest einen Extraktionsbeutel verbunden und zum Verformen des zumindest einen Extraktionsbeutels unter Verengung der zumindest einen Öffnung ausgebildet ist. Zudem weist dieser Extraktionsbeutel eine oder mehrere Tüllen wie im Vorhergehenden beschrieben auf und weist zusammen mit den beschriebenen Ausgestaltungen der Tülle einen eigenständigen erfinderischen Gehalt auf. Die Extraktionsvorrichtung gemäß diesem Aspekt der Erfindung umfasst also beispielsweise eine Tülle, welche eine mit dem zumindest einen Extraktionsbeutel verbundene und einen Tülleninnenraum der wenigstens einen Tülle zumindest bereichsweise umgrenzende Tüllenwandung aufweist, wobei der Tülleninnenraum über wenigstens eine am Extraktionsbeutel ausgebildete Durchgangsöffnung in den wenigstens einen Beutelinnenraum mündet und wobei zumindest eine mit der Tüllenwandung verbundene Lasche sowie wenigstens ein, durch eine Laschenöffnung der zumindest einen Lasche geführtes Verengungselement, welches zum Verformen der zumindest einen Tüllenwandung und bereichsweisen Verengen des Tülleninnenraums ausgebildet ist, vorgesehen sind.

In einer weiteren vorteilhaften Weiterbildung der Erfindung umfasst die Extraktionseinrichtung zumindest ein Faserelement, welches an zumindest einer an einem Beutelboden des zumindest einen Extraktionsbeutels angeordneten Bodenlasche befestigt ist. Dies ist von Vorteil, da das Faserelement zusammen mit dem Extraktionsbeutel aufgerollt werden kann und ein Entrollen des Extraktionsbeutels erleichtern kann. Dadurch kann ebenfalls Zeit beim medizinischen Eingriff gespart werden. Der Vorteil des Faserelements besteht also mit anderen Worten darin, dass durch dessen Anordnung an der Bodenlasche beispielsweise ein vereinfachtes Entrollen des Extraktionsbeutels beim medizinischen Eingriff erfolgen kann. Hierzu kann das Faserelement beispielsweise mit einer Zange gegriffen und der Extraktionsbeutel an dessen Bodenlasche über das Faserelement bewegt werden. Dies kann insbesondere dann von Vorteil sein, wenn der Extraktionsbeutel eine glatte Oberfläche aufweist, welche ein Greifen des Extraktionsbeutels, beispielsweise mittels der Zange, erschwert. Besonders einfach kann das Faserelement gegriffen werden, wenn das an der Bodenlasche befestigte Faserelement bereichsweise von der Bodenlasche abragt. An der Bodenlasche kann bevorzugt ein Loch vorgesehen sein, in welches das zumindest eine Faserelement eingeführt und befestigt, beispielsweise verknotet, verklebt oder verschweißt sein kann. Das Loch an der Bodenlasche trägt nicht zu einer unerwünschten Perforation des Extraktionsbeutels bei, sodass der Extraktionsbeutel dicht bleibt und ein Austreten von Gewebe, Gewebeflüssigkeit und/oder Blut aus dem Beutelinnenraum vermieden werden kann.

Ein weiterer Aspekt der Erfindung betrifft eine Extraktionseinrichtung zur chirurgischen Entnahme von Gewebe, mit zumindest einem Extraktionsbeutel, welcher wenigstens eine Öffnung zum Einführen des Gewebes in wenigstens einen Beutelinnenraum des zumindest einen Extraktionsbeutels aufweist und mit zumindest einer Zugvorrichtung, welche mit dem zumindest einen Extraktionsbeutel verbunden und zum Verformen des zumindest einen Extraktionsbeutels unter Verengung der zumindest einen Öffnung ausgebildet ist. Zudem weist dieser Extraktionsbeutel zumindest ein Faserelement auf, welches an zumindest einer an einem Beutelboden des zumindest einen Extraktionsbeutels angeordneten Bodenlasche befestigt ist. Dies ist von Vorteil, da das Faserelement zusammen mit dem Extraktionsbeutel aufgerollt werden kann und ein Entrollen des Extraktionsbeutels erleichtern kann. Dieser Extraktionsbeutel weist ebenfalls einen eigenständigen erfinderischen Gehalt auf.

Ein zweiter Aspekt der Erfindung betrifft eine Applikationsvorrichtung mit einer Extraktionseinrichtung gemäß dem ersten Aspekt der Erfindung, und mit einem Applikator, welcher einen Applikatorinnenraum aufweist, in welchem die Extraktionseinrichtung in aufgerolltem Zustand aufgenommen ist. Anhand des Applikators kann die Extraktionseinrichtung durch besonders kleine Körperöffnungen in den Körper eines Patienten eingeführt werden. Der Applikator kann bevorzugt rohrförmig ausgebildet sein und beispielsweise einen Hohlzylinderquerschnitt aufweisen. Dadurch kann der Applikator auch besonders aufwandsarm hergestellt werden. Die im Zusammenhang mit der erfindungsgemäßen Extraktionseinrichtung gemäß dem ersten Aspekt der Erfindung vorgestellten Merkmale sowie deren Vorteile gelten entsprechend für die erfindungsgemäße Applikationsvorrichtung gemäß dem zweiten Aspekt der Erfindung und umgekehrt.

In einer vorteilhaften Weiterbildung der Erfindung sind der Beutelboden und die zumindest eine Bodenlasche in aufgerolltem Zustand der Extraktionseinrichtung von einer, den Applikatorinnenraum begrenzenden Applikatorwandung des Applikators beabstandet. Dies erleichtert in vorteilhafter Weise ein kontrolliertes Herausführen, beispielsweise in Form eines Herausschiebens, der Extraktionseinrichtung aus dem Applikatorinnenraum.

In einer weiteren vorteilhaften Weiterbildung der Erfindung ist das zumindest eine Faserelement im aufgerollten Zustand der Extraktionseinrichtung zumindest bereichsweise von dem Extraktionsbeutel umgeben. Dies ist von Vorteil, da das Faserelement dadurch in den Extraktionsbeutel eingerollt sein kann, sodass die Extraktionseinrichtung unter einem Gleitkontakt zwischen der Beutelwand und der Applikatorwandung reibungsarm aus dem Applikatorinnenraum herausgeschoben werden kann, ohne dass das Herausschieben durch das Faserelement erschwert oder behindert wird.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: eine schematische Draufsicht auf eine Vorderseite einer Extraktionseinrichtung;
- Fig. 2: eine schematische Draufsicht auf eine Rückseite der Extraktionseinrichtung;
- Fig. 3: eine schematische Perspektivansicht zweier miteinander gekoppelter Zugvorrichtungsenden einer Zugvorrichtung der Extraktionseinrichtung;
- Fig. 4: eine schematische Seitenansicht einer Tülle der Extraktionseinrichtung;
- Fig. 5: eine vergrößerte Darstellung eines in Fig. 4 umrandeten Bereichs A;
- Fig. 6: eine schematische Seitenansicht einer weiteren Tülle der Extraktionseinrichtung;
- Fig. 7: eine schematische Perspektivansicht einer Applikationsvorrichtung;
- Fig. 8: eine schematische Schnittdarstellung der Applikationsvorrichtung;
- Fig. 9-11: verschiedene schematische Seitenansichten eines Verengungselements, welche eine Herstellung eines Knotens am Verengungselement zeigen.

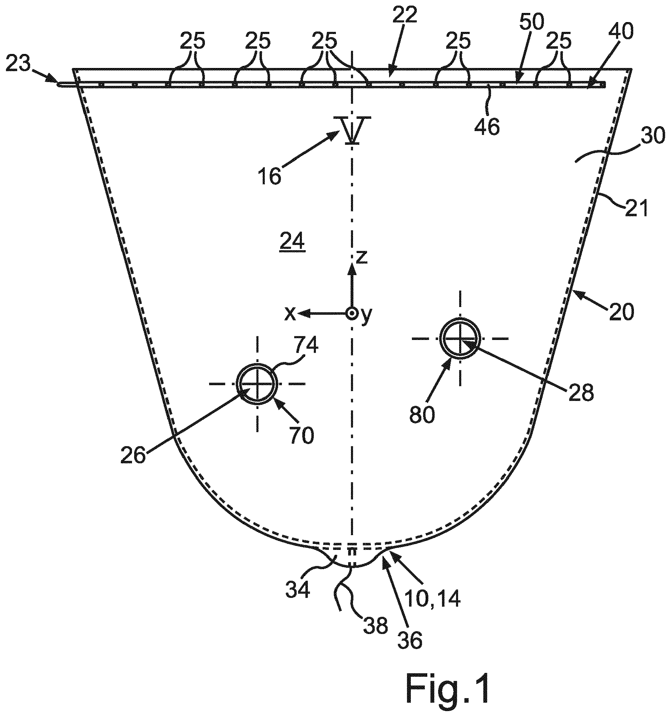

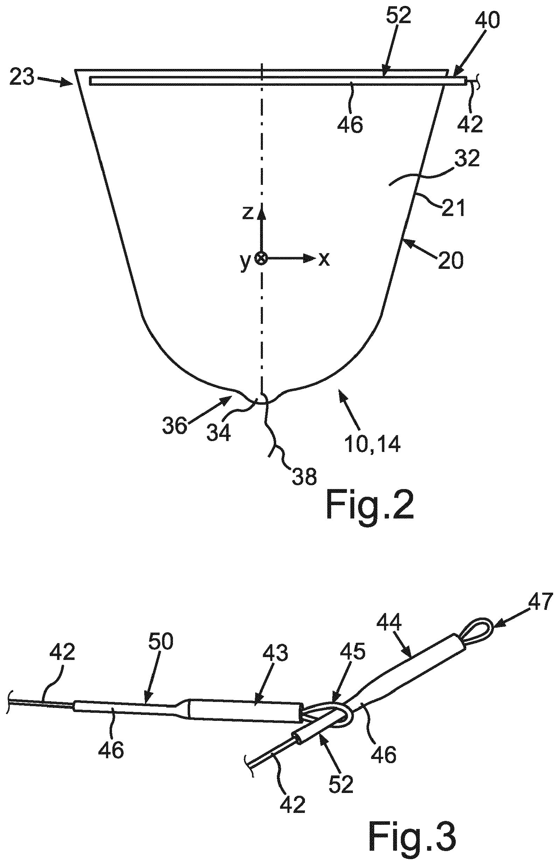

Fig. 1 zeigt in einer schematischen Draufsicht eine Vorderseite 30 und Fig. 2 eine Rückseite 32 einer sterilen Extraktionseinrichtung 10, welche zur chirurgischen Entnahme von Gewebe, beispielsweise bei einer Endoskopie, dient.

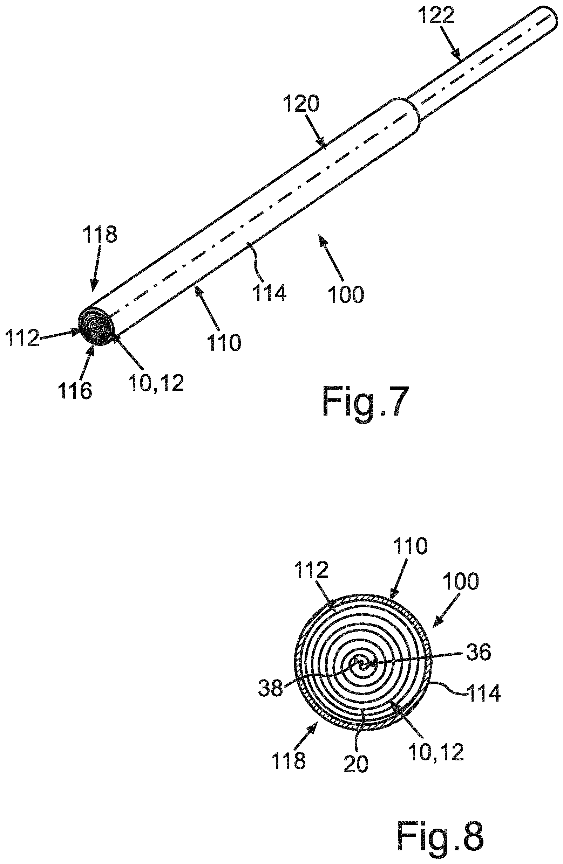

In Fig. 1 und Fig. 2 sind jeweils auf die Extraktionseinrichtung 10 bezogene Koordinatensysteme angegeben, welche durch eine Längserstreckungsrichtung x, durch eine Quererstreckungsrichtung y sowie durch eine Hocherstreckungsrichtung z der Extraktionseinrichtung 10 definiert sind. Eine Pfeilrichtung eines die Hocherstreckungsrichtung z verdeutlichenden Pfeils entspricht vorliegend einer Wickelrichtung in welcher die Extraktionseinrichtung 10 von einem in Fig. 1 und Fig. 2 gezeigten, abgerollten Zustand 14 in einen, in Fig. 7 und Fig. 8 schematisch dargestellten, aufgerollten Zustand 12 zusammengerollt und damit aufgewickelt werden kann. Im aufgerollten Zustand 12 kann die Extraktionseinrichtung 10 in einem Applikatorinnenraum 112 eines ebenfalls in Fig. 7 und Fig. 8 schematisch dargestellten Applikators 110 aufgenommen werden. Die Extraktionseinrichtung 10 und der Applikator 110 bilden dann gemeinsam eine Applikationsvorrichtung 100.

Die Extraktionseinrichtung 10 umfasst einen Extraktionsbeutel 20, welcher eine Öffnung 22 zum Einführen des Gewebes in einen Beutelinnenraum 24 des Extraktionsbeutels 20 aufweist. Der Beutelinnenraum 24 ist durch eine Beutelwand 21 des Extraktionsbeutels 20 zumindest bereichsweise begrenzt. Die vorliegend aus Kunststoff gebildete Beutelwand 21 kann vorzugsweise zumindest bereichsweise, insbesondere vollständig, transparent und damit durchsichtig ausgebildet sein.

Ebenso umfasst die Extraktionseinrichtung 10 ein Faserelement 38, welches an einer an einem Beutelboden 36 des Extraktionsbeutels 20 angeordneten Bodenlasche 34 befestigt ist.

Die Extraktionseinrichtung 10 umfasst des Weiteren eine Zugvorrichtung 40, welche mit dem Extraktionsbeutel 20 verbunden und zum Verformen des Extraktionsbeutels 20 unter Verengung der Öffnung 22 ausgebildet ist.

Die Zugvorrichtung 40 umfasst voneinander verschiedene Markierungen 50, 52 zur Kennzeichnung der verschiedenen Seiten 30, 32 (Vorderseite 30, Rückseite 32) des zumindest einen Extraktionsbeutels 20.

Die Zugvorrichtung 40 umfasst ein Zugelement 42 und eine Ummantelung 46, welche das Zugelement 42 umgibt, wie deutlich in Fig. 3 erkennbar ist. Das Zugelement 42 ist dabei als Draht, insbesondere als Nitinol-Draht, ausgebildet. In Fig. 3 sind zwei Vorrichtungsenden, nämlich ein erstes Vorrichtungsende 43 und ein zweites Vorrichtungsende 44 der Zugvorrichtung 40 gezeigt. An dem ersten Vorrichtungsende 43 weist das Zugelement 42 eine erste Elementschlaufe 45 auf, durch welche das zweite Vorrichtungsende 44 hindurchgeführt ist. Dadurch kann das Verformen des Extraktionsbeutels 20 unter Verengung der Öffnung 22 besonders einfach erfolgen, indem an dem zweiten Vorrichtungsende 44 angezogen und dadurch das Zugelement 42 mit dessen Ummantelung 46 zugezogen wird. An dem zweiten Vorrichtungsende 44 weist das Zugelement 42 eine zweite Elementschlaufe 47 auf, wodurch das zweite Vorrichtungsende 44 besonders sicher gegriffen werden kann. Das Zugelement 42, welches als Zugdraht ausgebildet sein kann, ist insgesamt derart gestaltet, dass die erste Elementschlaufe 45, welche ein erstes Elementende des Zugelements 42 bildet, die zweite Elementschlaufe 47, welche ein zweites Elementende des Zugelements 42 bildet, führen kann. Die Elementschlaufen 45, 47 können vorzugsweise derart dimensioniert sein, dass das zweite Vorrichtungsende 44 und damit die zweite Elementschlaufe 47 nicht durch die erste Elementschlaufe 45 hindurchrutschen kann. Dies ermöglicht eine besonders sichere Handhabung der Zugvorrichtung 40.

Die Ummantelung 46 kann mit den voneinander verschiedenen Markierungen 50, 52 versehen sein, um dadurch eine vereinfachte Orientierung des Extraktionsbeutels 20 bei der chirurgischen Entnahme des Gewebes zu ermöglichen. So kann beispielsweise die Ummantelung 46 auf der Vorderseite 30 des Extraktionsbeutels 20 mit einer ersten Markierung 50 der voneinander verschiedenen Markierungen 50, 52 und auf der Rückseite 32 des Extraktionsbeutels 20 mit einer zweiten Markierung 52 der voneinander verschiedenen Markierungen 50, 52 versehen sein. Denkbar ist allgemein auch, dass das Zugelement 42 mit den voneinander verschiedenen Markierungen 50, 52 versehen ist. Die voneinander verschiedenen Markierungen 50, 52 können beispielsweise unterschiedlich gefärbt sein. So kann beispielsweise die erste Markierung 50 gelb gefärbt und die zweite Markierung 52 schwarz gefärbt sein, wodurch eine besonders deutliche Unterscheidung der Vorderseite 30 von der Rückseite 32 ermöglicht ist.

An einem die Öffnung 22 begrenzenden Randbereich 23 des Extraktionsbeutels 20 ist vorliegend zumindest an dessen Vorderseite 30 eine Mehrzahl an Durchtrittsöffnungen 25 vorgesehen. Die Durchtrittsöffnungen 25 sind in Umfangsrichtung entlang dem Randbereich 23 verteilt. Die Zugvorrichtung 40 ist vorliegend in die Durchtrittsöffnungen 25 eingeführt, wodurch die Zugvorrichtung 40 besonders verliersicher an dem Extraktionsbeutel 20 geführt ist.

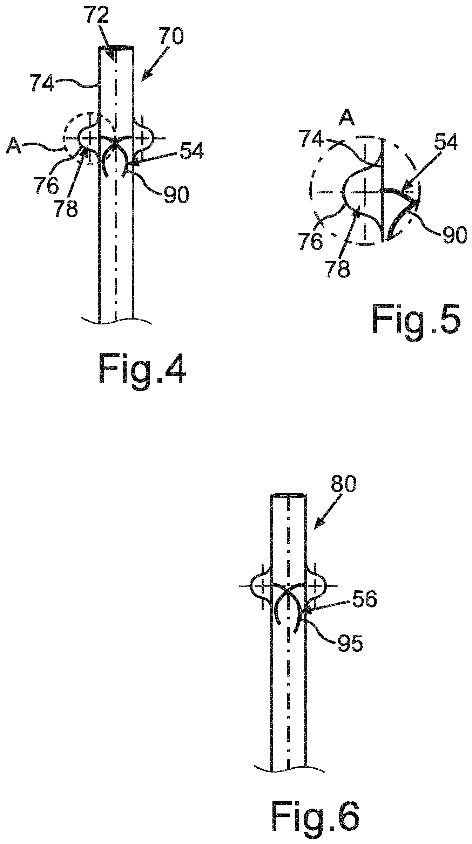

Die Extraktionseinrichtung 10 umfasst zudem eine, in Fig. 4 vergrößert dargestellte, erste Tülle 70 und eine, in Fig. 6 vergrößert dargestellte, zweite Tülle 80. Über die Tüllen 70, 80 können während des Eingriffs etwaige Werkzeuge in den Beutelinnenraum 24 eingeführt werden, um beispielsweise das im Beutelinnenraum 24 enthaltene Gewebe zu bearbeiten oder zu untersuchen. Nachfolgend wird vor allem der Aufbau der ersten Tülle 70 beschrieben, die zweite Tülle 80 kann jedoch einen vergleichbaren, insbesondere identischen, Aufbau aufweisen.

Die erste Tülle 70 weist eine mit dem Extraktionsbeutel 20 verbundene, beispielsweise verschweißte, und einen Tülleninnenraum 72 der ersten Tülle 70 bereichsweise umgrenzende Tüllenwandung 74 auf. Der Tülleninnenraum 72 mündet über eine am Extraktionsbeutel 20 ausgebildete Durchgangsöffnung 26 in den Beutelinnenraum 24. Ein der zweiten Tülle 80 zugeordneter, zweiter Tülleninnenraum mündet hingegen über eine weitere, am Extraktionsbeutel 20 ausgebildete Durchgangsöffnung 28 in den Beutelinnenraum 24. Wie in Fig. 1 erkennbar ist, sind die Durchgangsöffnungen 26, 28 an der Vorderseite 30 des Extraktionsbeutels 20 angeordnet.

Zudem ist eine, mit der Tüllenwandung 74 verbundene Lasche 76 der Extraktionseinrichtung 10 sowie ein, durch eine Laschenöffnung 78 der Lasche 76 geführtes Verengungselement 90 der Extraktionseinrichtung 10 vorgesehen. Die Lasche 76 ist vor allem in Fig. 5 deutlich erkennbar, welche einen in Fig. 4 ringförmig umrandeten Bereichs A in vergrößerter Darstellung zeigt.

Das Verengungselement 90 ist zum Verformen der Tüllenwandung 74 und bereichsweisen Verengen des Tülleninnenraums 72 ausgebildet. Die Tüllenwandung 74 ist zwischen dem Tülleninnenraum 72 und dem Verengungselement 90 angeordnet. Dabei trennt die Tüllenwandung 74 den Tülleninnenraum 72 von dem Verengungselement 90, um eine Kontamination des Verengungselements 90 mit in dem Beutelinnenraum 24 enthaltenem Gewebe zu vermeiden.

Das Verengungselement 90 ist vorliegend als Faden ausgebildet. Alternativ dazu könnte das Verengungselement 90 jedoch auch als Seil oder als Draht ausgebildet sein.

An der zweiten Tülle 80 ist in vergleichbarer Weise ein zweites Verengungselement 95 angeordnet, welches eine vergleichbare Funktion wie das Verengungselement 90 erfüllt. So kann insgesamt der zweite Tülleninnenraum anhand des zweiten Verengungselements 95 verengt werden, ebenso wie der (erste) Tülleninnenraum 72 anhand des ersten Verengungselements 90 verengt werden kann. Die zweite Tülle 80 der Extraktionseinrichtung 10, kann also durch das zweite Verengungselement 95 verformt und bereichsweise verengt werden.

Um die Orientierung des Extraktionsbeutels 20 zusätzlich zu vereinfachen, weisen das zweite Verengungselement 95 und das Verengungselement 90 voneinander verschiedene Markierungen 54, 56 auf. Die im Zusammenhang mit der ersten Markierung 50 und der zweiten Markierung 52 vorgestellten Merkmale sowie deren Vorteile können auch entsprechend für die Markierungen 54, 56 gelten. So können beispielsweise sämtliche Markierungen 50, 52, 54, 56 unterschiedlich gefärbt, zusätzlich oder alternativ unterschiedlich strukturiert und zusätzlich oder alternativ unterschiedlich beschriftet sein, um nur einige Beispiele zu nennen.

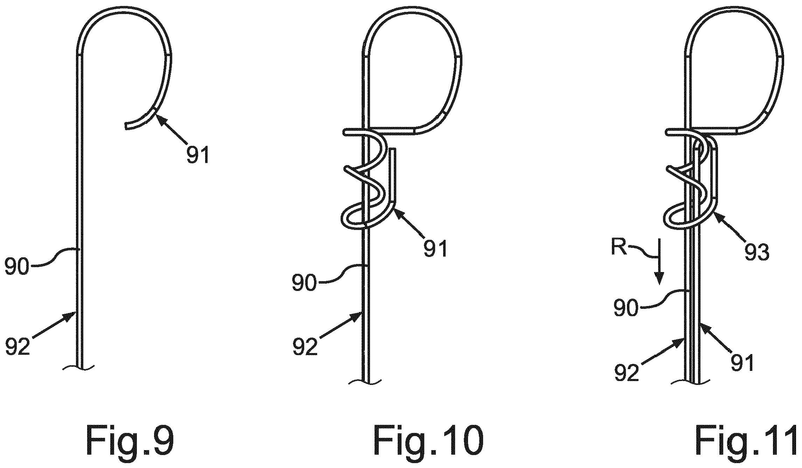

Das Verengungselement 90 kann allgemein verknotet werden, wie in Zusammenschau von Fig. 9, Fig. 10 und Fig. 11 erkennbar ist. An einem ersten Verengungselementbereich 91 des Verengungselements 90 ist in Fig. 11 ein Knoten 93 geknüpft, wobei der Knoten 93 derart geknüpft ist, dass der Knoten 93 verschiebbar an einem zweiten Verengungselementbereich 92 des Verengungselements 90 aufgenommen ist. Die Tüllenwandung 74 kann dann durch eine, durch einen Pfeil verdeutlichte Relativverschiebung R zwischen dem Knoten 93 und dem zweiten Verengungselementbereich 92 anhand des Verengungselements 90 verformt und dabei der Tülleninnenraum 72 zumindest bereichsweise verengt werden.

Fig. 7 und Fig. 8 zeigen jeweils die Applikationsvorrichtung 100 mit der Extraktionseinrichtung 10, und mit dem Applikator 110, welcher den Applikatorinnenraum 112 aufweist, in welchem die Extraktionseinrichtung 10 in aufgerolltem Zustand 12 aufgenommen ist.

Der Beutelboden 36 und die Bodenlasche 34 sind in aufgerolltem Zustand 12 der Extraktionseinrichtung 10 von einer, den Applikatorinnenraum 112 begrenzenden Applikatorwandung 114 des Applikators 110 beabstandet. Der Randbereich 23 kann ebenso wie die Zugvorrichtung 40 an der Applikatorwandung 114 anliegen, sodass bereits beim Herausbewegen (Herausschieben und/oder Herausziehen) der Extraktionseinrichtung 10 und noch in deren aufgerolltem Zustand 12 erkennbar ist, wo sich die Vorderseite 30 und die Rückseite 32 des Extraktionsbeutels 20 befinden. Wird also die Applikationsvorrichtung 100 beim chirurgischen Eingriff in den Körperinnenraum eingebracht, so kann besonders frühzeitig bereits beim Herausbewegen der Extraktionseinrichtung 10 aus dem Applikatorinnenraum 112 des Applikators 110 erkannt werden, wo sich die Vorderseite 30 und Rückseite 32 des Extraktionsbeutels 20 befinden, selbst wenn sich die Extraktionseinrichtung 10 noch nicht in deren abgerolltem Zustand 14 (siehe Fig. 1 und Fig. 2) befindet. Dies ermöglicht in besonders vorteilhafter Weise bereits beim Abrollen (entgegen der Pfeilrichtung des die Hocherstreckungsrichtung z verdeutlichenden Pfeils), also beim Übergang vom aufgerollten Zustand 12 in den abgerollten Zustand 14 eine korrekte Orientierung und Ausrichtung der Extraktionseinrichtung 10 bzw. des Extraktionsbeutels 12 vorzunehmen, wodurch eine erhebliche Zeitersparnis erzielt werden kann.

Dabei liegen der Beutelboden 36 und die Bodenlasche 34 zumindest im Wesentlichen im Bereich einer, hier nicht weiter gezeigten Mittelachse des Applikators 110, welcher vorliegend zwei Applikatorrohre 120, 122 umfasst, wobei das Applikatorrohr 122 in das Applikatorrohr 120 eingeführt ist. Das Applikatorrohr 120 bildet dabei ein Außenrohr, wohingegen das Applikatorrohr 122 ein im Applikatorrohr 120 aufgenommenes Innenohr bildet. Durch ein relatives Verschieben der Applikatorrohre 120, 122 zueinander kann die Extraktionseinrichtung 10 zumindest bereichsweise aus dem Applikatorinnenraum 112 herausgeschoben werden.

Das Faserelement 38 ist darüber hinaus im aufgerollten Zustand 12 der Extraktionseinrichtung 10 zumindest bereichsweise von dem Extraktionsbeutel 20 umgeben. Dadurch kann sich das Faserelement 38 im aufgerollten Zustand 12 der Extraktionseinrichtung 10 zumindest im Wesentlichen im Bereich einer Mittelachse des Applikators 110 befinden, wie in Fig. 8 angedeutet ist. Der Applikator 110 weist eine Applikatoröffnung 116 auf, welche sich an einem Ende 118 des Applikators 110 befindet und in den Applikatorinnenraum 112 mündet. Über die Applikatoröffnung 116 kann die sich im aufgerollten Zustand 12 befindende Extraktionseinrichtung 10 aus dem Applikatorinnenraum 112 herausgeschoben werden.

Zusammenfassend kann beispielsweise eine Ausgestaltung der Extraktionseinrichtung 10 vorgesehen sein, bei welcher das Zugelement 42 als Nitinol-Draht ausgebildet sein kann und die Ummantelung 46 zweifarbig ausgebildet sein kann. Die Markierungen 50, 52 können also verschiedenfarbig sein, wobei beispielsweise die erste Markierung 50 gelb und die zweite Markierung 52 schwarz ausgebildet sein kann. Das Zugelement 42 bzw. die Zugvorrichtung 40 verläuft im Bereich der Öffnung 22, welche auch als Hauptöffnung des Extraktionsbeutels 20 bezeichnet werden kann, um die Vorderseite 30 (Oberseite) bzw. Rückseite 32 (Unterseite) des Extraktionsbeutels 20 zu kennzeichnen. Zusätzlich kann eine Bedruckung 16, beispielsweise in Form eines Buchstabens (in Fig. 1: Buchstabe "V") oder eines Schriftzugs, beispielsweise in Hocherstreckungsrichtung z unterhalb der Zugvorrichtung 40 an der Beutelwand 21 des Extraktionsbeutels 20 angeordnet sein. Die Bedruckung 16 kann derart ausgebildet sein, dass diese nur lesbar ist, wenn der Extraktionsbeutel 20 eine korrekte Orientierung aufweist. Dies erleichtert beispielsweise die Ausrichtung und Platzierung der Extraktionseinrichtung 10, wenn diese beispielsweise im Rahmen eines chirurgischen Eingriffs im Abdomen eines Patienten angeordnet ist.

Die Verengungselemente 90, 95, können vorliegend als jeweilige Fäden mit den unterschiedlichen Markierungen 54, 56 ausgebildet sein. Die Verengungselemente 90, 95 dienen zum Verengen und Verknoten der jeweiligen Tüllen 70, 80. Im Gegensatz zu konventionellen Vorrichtungen sind die Verengungselemente 90, 95 vorliegend nicht über Löcher in den jeweiligen Tüllen 70, 80 befestigt, sodass auch beispielsweise keine Zellen oder Blut (von dem Gewebe) aus dem Extraktionsbeutel 20 entweichen kann. Im Gegensatz zu konventionellen Vorrichtungen sind die Verengungselemente 90, 95 bei der vorliegenden Extraktionseinrichtung 10 hingegen an den extra an den Tüllen 70, 80 angebrachten Laschen 76 aufgenommen. Die Tüllen 70, 80 sind derart ausgestaltet, dass diese in Richtung des Beutelinnenraums 24 und damit nach innen umstülpbar sind, um etwaige, kontaminierte Randzonen der Tüllen 70, 80 in den geschützten Beutelinnenraum 24 einbringen und dort anhand der Verengungselemente 90, 95 verschließen zu können.

Der Knoten 93 kann bevorzugt als vorgelegter, verschiebbarer Knoten ausgebildet sein und zum einfachen, sicheren Verschluss der Tüllen 70, 80 an den jeweiligen Verengungselemente 90, 95 vorgesehen sein. Zudem ist aufgrund der Verschiebbarkeit ein einfaches rasches Öffnen des Knotens 93 im Bedarfsfall ermöglicht.

Das Faserelement 38 kann als zusätzlicher Faden ausgebildet und an der Bodenlasche 34 des Beutelbodens 36 und damit an einem unteren Beutelende des Extraktionsbeutels 20 angebracht sein. Das Faserelement 38 kann mit dem Extraktionsbeutel 20 aufgerollt werden und derart im Applikatorinnenraum 112 des Applikators 110 angeordnet werden, dass sich das Faserelement 38 zumindest im Wesentlichen im Bereich der Mittelachse des Applikators 110 befindet. Ein Entrollen der Extraktionseinrichtung 10 kann - nach dem Herausschieben der Extraktionseinrichtung 10 aus dem Applikatorinnenraum 112 - auf einfache Art und Weise durch ein Halten des Extraktionsbeutels 20 an der Beutelwand 21 im Bereich der Öffnung 22, also am Randbereich 23, beispielsweise mit einer Fasszange sowie ein Ziehen am Faserelement 38 mit einer zweiten Zange erfolgen. Dadurch gestaltet sich das Entrollen der Extraktionseinrichtung 10 und damit des Extraktionsbeutels 20 besonders unkompliziert und schnell.

## Patentansprüche

1. Extraktionseinrichtung (10) zur chirurgischen Entnahme von Gewebe, mit zumindest einem Extraktionsbeutel (20), welcher wenigstens eine Öffnung (22) zum Einführen des Gewebes in wenigstens einen Beutelinnenraum (24) des zumindest einen Extraktionsbeutels (20) aufweist und mit zumindest einer Zugvorrichtung (40), welche mit dem zumindest einen Extraktionsbeutel (20) verbunden und zum Verformen des zumindest einen Extraktionsbeutels (20) unter Verengung der zumindest einen Öffnung (22) ausgebildet ist,
**dadurch gekennzeichnet, dass**
die zumindest eine Zugvorrichtung (40) voneinander verschiedene Markierungen (50, 52) zur Kennzeichnung verschiedener Seiten (30, 32) des zumindest einen Extraktionsbeutels (20) umfasst.

2. Extraktionseinrichtung (10) nach Anspruch 1,
**dadurch gekennzeichnet, dass**
die zumindest eine Zugvorrichtung (40) wenigstens ein Zugelement (42) und wenigstens eine Ummantelung (46) umfasst, welche das zumindest eine Zugelement (42) umgibt.

3. Extraktionseinrichtung (10) nach Anspruch 2,
**dadurch gekennzeichnet, dass**
die wenigstens eine Ummantelung (46) und/oder das wenigstens eine Zugelement (42) mit den voneinander verschiedenen Markierungen (50, 52) versehen ist.

4. Extraktionseinrichtung (10) nach Anspruch 2 oder 3,
**dadurch gekennzeichnet, dass**
das zumindest eine Zugelement (42) als Draht, insbesondere Nitinol-Draht, ausgebildet ist.

5. Extraktionseinrichtung (10) nach einem der vorhergehenden Ansprüche,
**dadurch gekennzeichnet, dass**
die verschiedenen Seiten (30, 32) eine Vorderseite (30) und eine Rückseite (32) des zumindest einen Extraktionsbeutels (20) umfassen.

6. Extraktionseinrichtung (10) nach einem der vorhergehenden Ansprüche,
**dadurch gekennzeichnet, dass**
die Extraktionseinrichtung (10) wenigstens eine Tülle (70) umfasst, welche eine mit dem zumindest einen Extraktionsbeutel (20) verbundene und einen Tülleninnenraum (72) der wenigstens einen Tülle (70) zumindest bereichsweise umgrenzende Tüllenwandung (74) aufweist, wobei der Tülleninnenraum (72) über wenigstens eine am Extraktionsbeutel (20) ausgebildete Durchgangsöffnung (26) in den wenigstens einen Beutelinnenraum (24) mündet und wobei zumindest eine mit der Tüllenwandung (74) verbundene Lasche (76) sowie wenigstens ein, durch eine Laschenöffnung (78) der zumindest einen Lasche (76) geführtes Verengungselement (90), welches zum Verformen der zumindest einen Tüllenwandung (74) und bereichsweisen Verengen des Tülleninnenraums (72) ausgebildet ist, vorgesehen sind.

7. Extraktionseinrichtung (10) nach Anspruch 6,
**dadurch gekennzeichnet, dass**
die Tüllenwandung (74) zwischen dem Tülleninnenraum (72) und dem wenigstens einen Verengungselement (90) angeordnet ist.

8. Extraktionseinrichtung (10) nach Anspruch 6 oder 7,
**dadurch gekennzeichnet, dass**
das Verengungselement (90) als Faden oder als Seil oder als Draht ausgebildet ist.

9. Extraktionseinrichtung (10) nach einem der Ansprüche 6 bis 8,
**dadurch gekennzeichnet, dass**
das wenigstens eine Verengungselement (90) zumindest einen ersten Verengungselementbereich (91) mit wenigstens einem Knoten (93) umfasst, wobei der wenigstens eine Knoten (93) verschiebbar an zumindest einem zweiten Verengungselementbereich (92) des wenigstens einen Verengungselements (90) aufgenommen ist und die zumindest eine Tüllenwandung (74) durch eine Relativverschiebung (R) zwischen dem wenigstens einen Knoten (93) und dem zumindest einen zweiten Verengungselementbereich (92) anhand des wenigstens einen Verengungselements (90) verformbar und dabei der Tülleninnenraum (72) zumindest bereichsweise verengbar ist.

10. Extraktionseinrichtung (10) nach einem der Ansprüche 6 bis 9,
**dadurch gekennzeichnet, dass**
die Extraktionseinrichtung (10) wenigstens eine zweite Tülle (80) umfasst, welche durch wenigstens ein zweites Verengungselement (95) verformbar und bereichsweise verengbar ist, wobei das wenigstens eine zweite Verengungselement (95) und das wenigstens eine Verengungselement (90) voneinander verschiedene Markierungen (54, 56) aufweisen.

11. Extraktionseinrichtung (10) nach einem der vorhergehenden Ansprüche,
**dadurch gekennzeichnet, dass**
die Extraktionseinrichtung (10) zumindest ein Faserelement (38) umfasst, welches an zumindest einer an einem Beutelboden (36) des zumindest einen Extraktionsbeutels (20) angeordneten Bodenlasche (34) befestigt ist.

12. Applikationsvorrichtung (100) mit einer Extraktionseinrichtung (10) nach einem der Ansprüche 1 bis 11, und mit einem Applikator (110), welcher einen Applikatorinnenraum (112) aufweist, in welchem die Extraktionseinrichtung (10) in aufgerolltem Zustand (12) aufgenommen ist.

13. Applikationsvorrichtung (100) nach Anspruch 12, wobei die Extraktionseinrichtung (10) nach Anspruch 11 ausgebildet ist,
**dadurch gekennzeichnet, dass**
der Beutelboden (36) und die zumindest eine Bodenlasche (34) in aufgerolltem Zustand (12) der Extraktionseinrichtung (10) von einer, den Applikatorinnenraum (112) begrenzenden Applikatorwandung (114) des Applikators (110) beabstandet sind.

14. Applikationsvorrichtung (100) nach Anspruch 12 oder 13,
**dadurch gekennzeichnet, dass**
das zumindest eine Faserelement (38) im aufgerollten Zustand (12) der Extraktionseinrichtung (10) zumindest bereichsweise von dem Extraktionsbeutel (20) umgeben ist.
